(19) Europäisches Patentamt
European Patent Office
Office européen des brevets

(11) **EP 3 801 650 B1**

(12) **FASCICULE DE BREVET EUROPEEN**

(45) Date de publication et mention de la délivrance du brevet:
**27.07.2022 Bulletin 2022/30**

(21) Numéro de dépôt: **19725383.4**

(22) Date de dépôt: **24.05.2019**

(51) Classification Internationale des Brevets (IPC):
***A61L 2/23*** *(2006.01)*

(52) Classification Coopérative des Brevets (CPC):
**A61L 2/23**

(86) Numéro de dépôt international:
**PCT/EP2019/063546**

(87) Numéro de publication internationale:
**WO 2019/224386 (28.11.2019 Gazette 2019/48)**

(54) **TABLETTE SOLIDE CHLOREE DESINFECTANTE ET COLOREE**

DESINFIZIERENDE UND GEFÄRBTE FESTE CHLORIERTE TABLETTE

DISINFECTANT AND DYED SOLID CHLORINATED TABLET

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.05.2018 FR 1854399**

(43) Date de publication de la demande:
**14.04.2021 Bulletin 2021/15**

(73) Titulaire: **EUROTAB OPERATIONS**
**42170 Saint-Just-Saint-Rambert (FR)**

(72) Inventeurs:
• **ARNAUD, Sandrine**
  **42680 SAINT MARCELLIN EN FOREZ (FR)**

• **POULY, Aloïs**
  **42000 SAINT ETIENNE (FR)**
• **VENET, Valérie**
  **69530 ORLIENAS (FR)**

(74) Mandataire: **Regimbeau**
**20, rue de Chazelles**
**75847 Paris Cedex 17 (FR)**

(56) Documents cités:
| EP-A1- 2 384 623 | WO-A1-2013/083612 |
| FR-A1- 2 212 427 | US-A- 4 668 475 |
| US-A1- 2008 135 062 | |

**Description**

La présente invention a pour objet une tablette solide destinée à la préparation d'une solution désinfectante ainsi que son procédé de préparation. La présente invention a également pour objet un procédé de préparation d'une solution désinfectante.

Dans le domaine de la désinfection, les tablettes comprenant un agent chloré, et notamment les tablettes de dichloroisocyanurate de sodium (DCCNA) sont bien connues pour leur pouvoir bactéricide en suspension ou en contact surface. Ces tablettes solides désinfectantes sont généralement introduites dans une solution aqueuse (ex. : l'eau), puis ladite solution aqueuse est utilisée pour désinfecter une surface. Par exemple, la tablette solide désinfectante peut être directement incorporée dans l'eau située au fond de la cuvette des toilettes WC (environ 1,5L d'eau). Le consommateur doit alors attendre un certain temps avant de pouvoir actionner la chasse d'eau afin de s'assurer que la désinfection a bien eut lieu. La tablette désinfectante peut également être incorporée dans un récipient d'eau (environ 5L par exemple) pour obtenir une eau de lavage destinée à la désinfection des surfaces et sols.

L'inconvénient de ces tablettes désinfectantes repose sur l'impossibilité pour le consommateur de savoir si la solution désinfectante est prête, c'est-à-dire si suffisamment de chlore actif a été libéré dans la solution. En effet, une solution est bactéricide si une quantité suffisante de chlore actif a été libérée. Par exemple, selon la norme NF EN 13697 (juin 2015), une solution est bactéricide de surface non poreuse si la concentration en DCCNA est de 1,02 g/L. Autre exemple, selon la norme NF EN 1276 (mars 2010), une solution est bactéricide pour la cuvette des toilettes WC si la concentration en DCCNA est aussi de 1,02 g/L. Le consommateur peut donc être amené à tenter de désinfecter une surface avec une solution qui n'est pas encore désinfectante car la concentration suffisante en chlore actif n'est pas encore atteinte.

La présente invention permet de remédier à ce problème en proposant un procédé de préparation d'une solution désinfectante durant lequel le consommateur observe la disparition ou un changement de coloration de la solution désinfectante indiquant que la concentration minimale efficace en chlore actif est atteinte.

A ce jour, aucune tablette solide désinfectante proposée sur le marché ne permet de colorer une solution aqueuse et d'observer un changement de coloration informant que la solution obtenue est bien désinfectante.

Des poudres ou tablettes solides désinfectantes comprenant des colorants sont connues. Par exemple US2008/135062, WO2013/083612 et FR2212427 décrivent des poudres ou tablettes solides désinfectantes pouvant comprendre un colorant. Cependant, aucune de ces poudres ou tablettes n'apporte d'information aux consommateurs quant à l'atteinte d'une concentration minimale efficace en chlore actif dans la solution désinfectante ni n'apporte une solution au problème de dégradation du colorant par le chlore.

Les inventeurs ont découvert de manière surprenante que l'utilisation d'une tablette comprenant un agent chloré et un colorant apte à colorer une solution aqueuse dans deux couches séparées, avec un ratio particulier agent chloré / colorant, permettait de préparer une solution désinfectante dans laquelle une variation de coloration est observée lorsque la quantité minimale efficace de l'agent chloré est atteinte.

La présente invention porte ainsi sur un procédé de préparation d'une solution désinfectante comprenant les étapes suivantes :

- Placer une tablette solide désinfectante dans une solution aqueuse, avantageusement l'eau ; et
- Attendre la disparition ou le changement de coloration de ladite solution aqueuse, indiquant que la concentration minimum efficace en chlore actif est atteinte,

caractérisé en ce que la tablette solide désinfectante comprend au moins une couche active comprenant un agent chloré en quantité suffisante pour obtenir une solution aqueuse désinfectante, et au moins une couche colorante comprenant un colorant apte à colorer la solution aqueuse et ne comprenant pas d'agent chloré, le ratio en poids agent chloré / colorant étant d'au moins 100/0,005, avantageusement compris de 100/0,005 à 100/10.

La présente invention porte également sur une tablette solide désinfectante telle que définie ci-dessus.

Dans le cadre de la présente invention, l'incorporation de la tablette solide selon l'invention dans la solution aqueuse entraine la libération du colorant et donc la coloration de l'eau. Le colorant étant facilement dégradé par le chlore, une certaine concentration en chlore actif libéré dans la solution aqueuse entraine ensuite la disparition ou la variation de la coloration de la solution aqueuse. Le ratio agent chloré/ colorant utilisé dans la tablette solide permet d'obtenir cette disparition ou variation de la coloration lorsque la quantité minimale efficace de chlore actif est libérée dans la solution aqueuse.

Afin d'obtenir une tablette stable et une coloration visible de la solution désinfectante, les inventeurs ont découvert que le colorant apte à colorer l'eau devait être introduit dans une couche différente de la couche comprenant l'agent chloré. Ceci est notamment dû au fait que le colorant est facilement dégradé par le chlore.

Dans le cadre de la présente invention, on entend par « solution désinfectante », toute solution ayant un pouvoir bactéricide, notamment un pouvoir bactéricide selon la norme NF EN 13697 (juin 2015) ou la norme NF EN 1276 (mars 2010).

Dans le cadre de la présente invention, on entend par « tablette solide », une composition compactée constituée d'un ensemble de particules groupées de manière compacte de façon à pouvoir être manipulée sans se désagréger. La tablette peut avoir une forme cylindrique avec une section ayant une forme quelconque telle qu'une forme circulaire, ovale, octogonale, ou parallélépipédique. Lorsque la section de la tablette est parallélépipédique, typiquement carrée ou rectangulaire, les coins de la tablette peuvent être courbes de manière à ce qu'ils soient moins cassants. Chaque couche a la même section de sorte que l'empilement qu'elles forment ensemble soit uniforme. La tablette peut également avoir toute autre forme géométrique (étoile, losange...). La tablette solide a une masse comprise entre 1 g et 250 g, avantageusement comprise entre 1 g et 100 g, plus avantageusement comprise entre 2 g et 50 g et notamment comprise entre 2 g et 20 g.

La tablette solide selon l'invention comprend au moins une couche active et au moins une couche colorante. La tablette solide selon l'invention peut donc être une tablette bicouche comprenant une couche active et une couche colorante. La tablette selon l'invention peut également être par exemple une tablette tricouche comprenant une couche active comprise entre deux couches colorantes.

Avantageusement, après introduction de la tablette solide désinfectante selon l'invention dans une solution aqueuse, la (ou les) couche(s) colorante(s) commence(nt) à se déliter plus rapidement que la (ou les) couche(s) active(s), permettant ainsi la libération du colorant apte à colorer l'eau avant la libération de l'agent chloré. Ceci a pour effet d'accentuer la disparition ou la variation de la coloration de la solution aqueuse une fois la quantité de chlore suffisante atteinte.

L'ordre de délitement des différentes couches de la tablette peut être contrôlé aisément par l'homme du métier. Par exemple en variant le poids ou la compaction de chacune des couches ou en variant la nature et/ou la teneur de l'agent permettant d'agir sur la dissolution de la tablette, tel qu'un agent effervescent, un agent de désintégration, un agent d'éclatement ou leurs mélanges.

Avantageusement, dans la tablette solide selon l'invention, le poids de la couche active est compris de 0,3 g à 30 g, avantageusement de 0,5 g à 30 g, avantageusement de 1 g à 25 g, plus avantageusement de 3 g à 15 g, et le poids de la couche colorante est compris de 0,1 g à 20 g, avantageusement de 0,3 g à 20 g, avantageusement de 0,3 g à 8 g, plus avantageusement de 2 g à 5 g.

Dans le cadre de la présente invention, on entend par « tablette solide désinfectante », toute tablette solide destinée à la préparation d'une solution désinfectante lorsqu'elle est incorporée dans une solution aqueuse.

Dans le cadre de la présente invention, la solution aqueuse utilisée pour préparation d'une solution désinfectante peut être toute solution aqueuse destinée au lavage et à la désinfection. Une telle solution aqueuse peut donc être par exemple de l'eau ou une solution aqueuse détergente et/ou blanchissante.

La tablette solide selon l'invention comprend au moins une couche active. Ladite couche active comprend au moins un agent chloré qui est avantageusement choisi parmi les dichloroisocyanurates, avantageusement l'agent chloré est le dichloroisocyanurate de sodium (DCCNA), le dichloroisocyanurate de potassium (DCCK), ou leur mélange, plus avantageusement le dichloroisocyanurate de sodium.

Les dichloroisocyanurates de sodium et de potassium peuvent être anhydres ou dihydratés.

Les dichloroisocyanurates de sodium et de potassium peuvent être sous forme amorphe ou cristalline.

En solution aqueuse, les dichloroisocyanurates s'hydrolysent pour former l'acide isocyanurique, de l'acide hypochloreux (HClO) et les sels correspondants, tels que l'hypochlorite de sodium (NaClO). Le degré d'hydrolyse et la teneur en ion hypochlorite dépendent du pH.

Selon l'invention, l'agent chloré est en quantité suffisante pour obtenir une solution désinfectante lorsque la tablette est incorporée dans une solution aqueuse. La teneur en agent chloré à incorporer dans la tablette solide peut être aisément déterminée par l'homme du métier en prenant en compte le poids de la tablette et la quantité de solution désinfectante désirée.

Avantageusement, lorsque l'agent chloré est le DCCNA, il est présent en quantité suffisante pour libérer 1,02 g/L de chlore actif dans une solution aqueuse. Cette teneur correspond à la teneur minimale pour obtenir une solution bactéricide selon les normes NF EN 13697 (juin 2015) et NF EN 1276 (mars 2010).

Avantageusement, la teneur en agent chloré dans la tablette solide selon l'invention est comprise entre 3 % et 98%, plus avantageusement entre 10 % et 70 %, en particulier entre 35 % et 65 %, exprimée en poids par rapport au poids total de la tablette solide.

Dans le cas d'une tablette solide bicouche, c'est-à-dire comprenant uniquement une couche active et une couche colorante, la quantité d'agent chloré comprise dans la couche active est avantageusement de 5 à 99 %, plus avantageusement de 30 à 85 %, de préférence de 50 à 80 %, exprimée en poids par rapport au poids total de la couche active.

Dans le cas d'une tablette solide comprenant plus de deux couches actives, la teneur en agent chloré pourra être répartie proportionnellement ou non dans les différentes couches actives.

Avantageusement, la couche active de la tablette solide selon l'invention comprend en outre un agent permettant d'agir sur la dissolution de la tablette, tel qu'un agent effervescent, un agent de désintégration, un agent d'éclatement ou leurs mélanges.

Au sens de la présente invention, on entend par « agent effervescent », un composé capable de libérer du gaz lorsqu'il est mis en contact avec de l'eau ou un autre liquide. L'agent effervescent permet plus particulièrement à la composition de se fragmenter et/ou se dissoudre rapidement dans l'eau ou un autre liquide, notamment dans une solution de lavage. L'agent effervescent peut en particulier être choisi parmi les acides organiques, leurs anhydrides ou leurs sels (tels que l'acide adipique, l'acide citrique, l'acide malique, l'acide tartrique, l'acide malonique, l'acide fumarique, l'acide maléique, l'acide succinique et leurs mélanges), les carbonates ou bicarbonates (tels que le carbonate ou bicarbonate de sodium, carbonate ou bicarbonate de potassium, carbonate ou bicarbonate de calcium, carbonate ou bicarbonate de magnésium et leur mélange), et un mélange de ceux-ci. Avantageusement, l'agent effervescent est un bicarbonate, en particulier le bicarbonate de sodium. Plus avantageusement, l'agent effervescent est un mélange de bicarbonate de sodium et d'un acide organique, notamment l'acide adipique ou l'acide citrique. De manière préféré, l'agent effervescent est un couple bicarbonate de sodium/acide adipique.

Au sens de la présente invention, on entend par « agent de désintégration », un composé solide soluble dans une solution aqueuse et se dissolvant rapidement en solution aqueuse, qui permet d'améliorer la perméabilité d'une composition solide lorsqu'elle est mise en contact avec une solution aqueuse. L'agent de désintégration peut en particulier être choisi parmi l'acide alginique, la carboxyméthylcellulose calcique, la carboxyméthylcellulose sodique, le dioxyde de silicium colloïdal, la croscarmellose telle que la croscarmellose sodique, la crospovidone, la gomme guar, le silicate de magnésium et d'aluminium, la méthylcellulose, la cellulose microcristalline, polyacrilin potassium, la cellulose en poudre, l'amidon prégélatinisé, l'alginate de sodium, l'amidon, le carboxyméthylamidon, l'amidon de maïs, l'amidon de pomme de terre, glycolate d'amidon sodique, le carbonate de calcium, la carboxyméthylcellulose réticulée, l'hydroxypropylcellulose faiblement substituée, la carmellose, la carmellose sodique, la carmellose calcique, la gélose, la caroube, karaya, pectine, la gomme adragante, la bentonite, la résine échangeuse de cations, la polyvinylpyrrolidone, la polyvinylpyrrolidone réticulée, les alginates, la polacrilline de potassium, la pulpe d'agrumes, le laurylsulfate de sodium, et leurs mélanges.

Au sens de la présente invention, on entend par « agent d'éclatement », un composé permettant d'augmenter la vitesse de délitement et/ou de dissolution d'une composition solide par gonflement, déformation plastique, effet de mèche, lorsqu'il entre en contact avec l'eau ou un autre liquide. L'agent d'éclatement peut en particulier être choisi parmi les amidons, modifiés ou non, les dérivés d'amidon, les celluloses, modifiées ou non, les dérivés de cellulose, les polyacrylates réticulés, les polyvinylpyrrolidones réticulés, les polysaccharides, les alginates tels que l'acide alginique et l'alginate de sodium, les dérivés de silicate d'aluminium, la silice et/ou les gommes et dérivés, et leurs mélanges.

Avantageusement, la (ou les) couche(s) active(s) de la tablette selon l'invention comprend entre 1 % et 95 % en poids, avantageusement entre 1 % et 90 % en poids, plus avantageusement entre 15 % et 70 % en poids, en particulier entre 20 % et 50 % en poids d'au moins un agent permettant d'agir sur la dissolution de la composition, tel qu'un agent effervescent, un agent de désintégration, un agent d'éclatement ou leurs mélanges, par rapport au poids total de la (ou les) couche(s) active(s).

Il est connu de l'homme du métier que la quantité d'agent(s) permettant d'agir sur la dissolution de la composition est adaptée en fonction du temps de dissolution à obtenir, du volume total de la solution aqueuse et de la quantité d'agent chloré présents dans la (ou les) couche(s) active(s).

De manière préférée, l'agent permettant d'agir sur la dissolution est au moins un agent effervescent, en particulier un couple d'agents effervescents. Dans ce cadre, l'agent effervescent sera en particulier choisi parmi les carbonates et bicarbonates, notamment le bicarbonate de sodium, l'acide citrique et l'acide adipique, et leurs mélanges ou un couple bicarbonate de sodium/acide adipique et bicarbonate de sodium/acide citrique. Plus avantageusement, l'agent permettant d'agir sur la dissolution est un mélange de bicarbonate de sodium et d'un acide organique, notamment l'acide adipique ou l'acide citrique. De manière préférée, l'agent permettant d'agir sur la dissolution est un couple bicarbonate de sodium/acide adipique ou bicarbonate de sodium/acide citrique.

Avantageusement, la couche active de la tablette solide selon l'invention peut également comprendre au moins un additif supplémentaire, tel qu'une charge inerte, un tensioactif, un lubrifiant, un agent de pastillage, un séquestrant, un parfum ou un colorant.

Lorsqu'une charge inerte est présente dans la couche active selon l'invention, elle peut par exemple être choisie parmi les zéolithes, les phyllosilicates, les carbonates de métal alcalin, par exemple le carbonate de sodium, les silicates de sodium, le chlorure de sodium, et leurs mélanges.

Lorsqu'un tensioactif est ajouté à la couche active, celui-ci permet d'apporter à la tablette une action détergente, en plus de l'action désinfectante mentionnée ci-dessus. Le tensioactif peut par exemple être choisi parmi les tensioactifs non ioniques, les tensioactifs anioniques, cationiques et amphotères et leurs mélanges, avantageusement les tensioactifs non ioniques, les tensioactifs anioniques, et leurs mélanges,. Comme tensioactifs non ioniques convenant à la tablette solide selon l'invention, on peut citer les alcools gras éthoxylés et/ou propoxylés, les copolymères d'oxyde d'éthylène et d'oxyde de propylène, les alkyl polyglucosides (AGP), et leurs mélanges. Comme tensioactifs anioniques convenant au produit solide selon l'invention, on peut citer les alkylbenzène sulfonates (ALS) (comme l'alkyl benzène sulfonate de sodium), les paraffines - ou alcanesulfonates, les alkyl sulfates (comme le sodium lauryl sulfoacétate), les alcool sulfates primaires, le α-oléofinsulfonate, les alkyl éther sulfates, les sulfosuccinates, les acylisothionates, les méthylester sulfonates, le savon, les sulfoalkylamides d'acides gras, les diglycolamides sulfates, les N-acyl amino acides et alkyl polyoxyéthylène carboxylates, et leurs mélanges.

Lorsqu'un lubrifiant est présent dans la couche active selon l'invention, il peut par exemple être choisi parmi les stéarates de métal alcalin, en particulier le stéarate de calcium et le stéarate de magnésium, les huiles minérales, les huiles végétales, et leurs mélanges. Comme huiles minérales convenant pour le lubrifiant de la tablette solide selon l'invention, on peut citer la paraffine, la vaseline, leurs dérivés et/ou leurs mélanges. Comme huiles végétales convenant pour le lubrifiant de la tablette solide selon l'invention, on peut citer l'huile de colza, l'huile de palme, l'huile de tournesol, l'huile d'arachide et leurs mélanges.

Par « agent séquestrant », on entend, au sens de la présente invention, un composé qui forme des ensembles chimiques avec des ions métalliques. Plus particulièrement, un séquestrant est un composé permettant de séquestrer des ions métalliques, notamment des ions calcium ou chlorure, d'une solution aqueuse. Le séquestrant ou agent séquestrant permet ainsi de baisser la dureté de l'eau ou le taux de calcaire dans l'eau. Lorsqu'un séquestrant est ajouté à la couche active, il peut par exemple être choisi parmi les zéolithes, les phyllosilicates, les carbonates de métal alcalin, les silicates de sodium, les polymères de polycarboxylate tels que les polyacrylates, les copolymères acrylique/maléique, les phosphonates acryliques, les polycarboxylates monomères tels que les citrates, les gluconates, les oxydisuccinates, les mono-, di- et tri-succinates de glycérol, les carboxyméthyloxysuccinates, les composés amino polycarboxyliques tels que les carboxyméthyloxymalonates d'acide méthylglycinediacétate (MGDA), l'acide diacétique glutamique (GLDA), l'imminodisuccinate (IDS), éthylènediamine disuccinate (EDDS), les dipicolinates, les nitrilotriacétates, les hydroxyéthyliminodi acétates, l'acide éthylène-diamine-tétraacétique (EDTA), l'acide nitrilotriacétique (NTA), orthophosphates, les métaphosphates, les pyrophosphates, les polyphosphates de métal alcalin tel que le triplolyphosphate de sodium, et leurs mélanges. L'agent séquestrant est de préférence choisi parmi le citrate de sodium, le phosphate de sodium, l'acide méthylglycinediacétique (MGDA), l'acide nitrilotriacétique (NTA), l'acide éthylène-diamine-tétraacétique (EDTA), l'acide diéthylène-triamine-pentaacétique ($H_5$DTPA ou Trilon DTPA), et l'acide hydroxyéthyléthylène-diamine-triacétique (HEDTA).

Lorsqu'un colorant est ajouté à la couche active, ce colorant n'est pas un colorant apte à colorer une solution aqueuse mais un colorant apte à colorer les poudres. Ainsi, le colorant permet d'apporter une coloration à la couche active mais n'aura aucun effet sur la coloration de la solution désinfectante.

La (ou les) couche(s) active(s) selon l'invention comprennent moins de 15% en poids, de préférence moins de 10% en poids, d'un additif supplémentaire, par rapport au poids total de la composition.

Avantageusement, dans la tablette solide selon l'invention, le poids de la (les) couche(s) active(s) est compris de 0,3 g à 30 g, avantageusement de 0,5 g à 30 g, plus avantageusement de 1 g à 25 g, plus avantageusement de 3 g à 15 g.

La tablette solide selon l'invention comprend en outre au moins une couche colorante. Ladite couche colorante comprend au moins un colorant apte à colorer une solution aqueuse, en particulier l'eau, et ne comprend pas d'agent chloré.

Dans la famille des colorants, il existe les pigments, les colorants aptes à colorer les poudres et les colorants aptes à colorer l'eau. Les pigments sont insolubles dans l'eau, ils ne sont donc pas aptes à colorer l'eau. Les colorants aptes à colorer les poudres permettent d'apporter une coloration aux poudres et donc aux différentes couches de la tablette solide selon l'invention. Cependant, ces colorants ne permettent pas de colorer l'eau (ou toute autre solution aqueuse) lorsque les poudres colorées sont plongées dans l'eau. Parmi les colorants aptes à colorer les poudres, on peut citer : Hansa yellow G042, PV past blue BG, et permanent orange G. Enfin, les colorants aptes à colorer l'eau (ou toute autre solution aqueuse), permettent d'apporter une coloration de la solution aqueuse dans laquelle une tablette les comprenant est plongée. Parmi les colorants aptes à colorer l'eau, on peut citer par exemple : Sanolin blue NBL, vert sanolin R-3GL, E102TATRAZINE LAKE, E33 BRILLIANT BLUE FCF LAKE, et leurs mélanges.

L'homme du métier sait distinguer les colorants de chacune de ces catégories. Par exemple, afin de déterminer si un colorant est apte à colorer l'eau (ou toute autre solution aqueuse), l'homme du métier pourra appliquer le test suivant : Dans 1 litre d'eau du robinet disperser 0,2 grammes de colorant à tester. Si aucune coloration de l'eau n'est observée, ce colorant n'est pas considéré comme apte à colorer l'eau. Si une faible coloration est observée et que le colorant n'est pas complètement soluble dans l'eau (présence d'un léger résidu), le colorant en question pourra être considéré comme apte à colorer l'eau mais ne sera pas un choix préférentiel. Si une coloration intense sans résidu est observée, le colorant est considéré comme apte à colorer l'eau et est préférentiellement utilisé dans le cadre de la présente invention.

Avantageusement, dans le cadre de la présente invention, le colorant apte à colorer l'eau est choisi dans le groupe consistant en Sanolin blue NBL, vert sanolin R-3GL, E102TATRAZINE LAKE, E33 BRILLIANT BLUE FCF LAKE, et leurs mélanges, avantageusement en Sanolin blue NBL, vert sanolin R-3GL, et leurs mélanges.

Selon l'invention, le colorant est en quantité suffisante pour obtenir une solution colorée lorsque la tablette est incorporée dans une solution aqueuse. La teneur en colorant à incorporer dans la tablette solide est telle que le ratio en poids agent chloré / colorant est d'au moins 100/0,005, avantageusement compris de 100/0,005 à 100/10, avantageusement de 100/0,005 à 100/5, avantageusement de 100/0,005 à 100/3, plus avantageusement de 100/0,01 à 100/1, plus avantageusement de 100/0,1 à 100/1, plus avantageusement de 100/0,2 à 100/0,75. De manière préférée, la teneur en colorant à incorporer dans la tablette solide est telle que le ratio en poids agent chloré / colorant est compris de 100/0,01 à 100/1, plus avantageusement de 100/0,1 à 100/1, plus avantageusement de 100/0,2 à 100/0,75. Ces dernières plages de ratio permettent d'obtenir une solution aqueuse initiale très colorée et une disparition totale de la coloration une fois la quantité suffisante de chlore actif obtenue. L'atteinte de la concentration efficace en chlore actif est ainsi très visible pour le consommateur.

Avantageusement, la teneur en colorant dans la tablette solide selon l'invention est comprise entre 0,002 % et 5 %, avantageusement entre 0,005 % et 5 %, plus avantageusement entre 0,01 % et 4 %, en particulier entre 0,01 % et 2 %, notamment entre 0,01 % et 1 % exprimée en poids par rapport au poids total de la tablette solide.

Dans le cas d'une tablette solide bicouche, c'est-à-dire comprenant uniquement une couche active et une couche colorante, la quantité de colorant comprise dans la couche colorante est avantageusement de 0,01 à 10 %, plus avantageusement de 0,1 à 5 %, de préférence de 0,1 à 2 %, exprimée en poids par rapport au poids total de la couche colorante.

Dans le cas d'une tablette solide comprenant plus de deux couches colorantes, la teneur en colorant pourra être répartie proportionnellement ou non dans les différentes couches colorantes ; l'important étant toujours de respecter le ratio agent chloré / colorant dans la tablette solide totale.

Avantageusement, la couche colorante de la tablette solide selon l'invention comprend en outre un agent permettant d'agir sur la dissolution de la tablette, tel qu'un agent effervescent, un agent de désintégration, un agent d'éclatement ou leurs mélanges. Les différents agents permettant d'agir sur la dissolution sont tels que définis plus haut dans le texte. Chacune des définitions est donc considérée comme reprise ici.

Avantageusement, la (ou les) couche(s) colorante(s) de la tablette selon l'invention comprend entre 0,1 % à 99,995 % en poids, avantageusement de 20 % à 99,995 % en poids, plus avantageusement de 60 % à 99,995 %, plus avantageusement de 80 % à 99,995 % en poids, d'au moins un agent permettant d'agir sur la dissolution de la composition, tel qu'un agent effervescent, un agent de désintégration, un agent d'éclatement ou leurs mélanges, par rapport au poids total de la (ou les) couche(s) colorante(s) .

Il est connu de l'homme du métier que la quantité d'agent(s) permettant d'agir sur la dissolution de la composition est adaptée en fonction du temps de dissolution à obtenir, du volume total de la solution aqueuse et de la quantité de colorant présents dans la (ou les) couche(s) colorante(s).

De manière préférée, l'agent permettant d'agir sur la dissolution est au moins un agent effervescent et au moins un agent de désintégration, en particulier un couple d'agents effervescents et un agent de désintégration. Dans ce cadre, l'agent de désintégration pourra être plus particulièrement la croscarmellose et l'agent effervescent sera en particulier choisi parmi les carbonates et bicarbonates, notamment le bicarbonate de sodium, l'acide citrique et l'acide adipique, et leurs mélanges ou un couple bicarbonate de sodium/acide adipique et bicarbonate de sodium/acide citrique. Plus avantageusement, l'agent permettant d'agir sur la dissolution est un mélange de bicarbonate de sodium et d'un acide organique, notamment l'acide adipique ou l'acide citrique. De manière préféré, l'agent permettant d'agir sur la dissolution est un couple bicarbonate de sodium/acide adipique ou bicarbonate de sodium/acide citrique.

Avantageusement, la couche colorante de la tablette solide selon l'invention peut également comprendre au moins un additif supplémentaire, tel qu'une charge inerte, un tensioactif, un lubrifiant, un agent de pastillage, un séquestrant, un parfum ou un colorant. Les additifs cités ici sont tels que définis plus haut dans le texte.

La (ou les) couche(s) colorante(s) selon l'invention comprennent moins de 40% en poids, de préférence moins de 10% en poids, d'un additif supplémentaire, par rapport au poids total de la composition.

Avantageusement, dans la tablette solide selon l'invention, le poids de la (les) couche(s) colorante(s) est compris de 0,1 à 20 g, avantageusement de 0,3 g à 20 g, avantageusement de 0,3 g à 8 g, plus avantageusement de 2 g à 5 g.

Un troisième objet de la présente invention porte sur un procédé de préparation d'une tablette solide telle que définie ci-dessus, comprenant les étapes suivantes :

a) mélanger les différents composants de la couche active de façon homogène, dont au moins un agent chloré en quantité suffisante pour libérer 1,02 g/L de chlore actif dans une solution aqueuse, pour former la composition active ;
b) pré-compacter la composition active, en particulier par compression directe, par exemple sur des presses rotatives ;
c) mélanger les différents composants de la couche colorante de façon homogène, dont au moins un colorant apte à colorer une solution aqueuse, pour former la composition colorante ;
d) pré-compacter la composition colorante sur la couche active préalablement formée à l'étape b), en particulier par compression directe, par exemple sur des presses rotatives ;
e) éventuellement renouveler les étapes a) à d) autant de fois que de couches nécessaires ; et

f) compacter l'ensemble des couches obtenue après l'étape d) ou e), en particulier par compression directe, par exemple sur des presses rotatives, pour obtenir une tablette solide,

l'ordre des étapes a)-b) et c)-d) pouvant éventuellement être inversé.

Avantageusement l'ordre des étapes a)-b) et c)-d) n'est pas inversé. Ainsi, la couche active est plus compactée que la couche colorante et se délitera donc moins rapidement que la couche colorante.

Avantageusement, les forces de pré-compaction utilisées sont comprises entre 5 kN et 200 kN, avantageusement entre 5 kN et 100 kN et plus avantageusement entre 10 kN et 80 kN. La force de compaction utilisée est en particulier comprise entre 20 kN et 1000 kN, avantageusement entre 40 kN et 600 kN et plus avantageusement entre 40 kN et 250 kN.

La composition compactée ainsi obtenue est résistante aux chocs.

Un autre objet de la présente invention porte sur l'utilisation d'une tablette solide telle que définie ci-dessus, pour préparer une solution désinfectante comprenant une concentration minimum efficace en chlore actif.

Notamment, la présente invention porte sur l'utilisation d'une tablette solide telle que définie ci-dessus, pour préparer une solution désinfectante de surface et/ou de sol, comprenant une concentration minimum efficace en chlore actif.

La présente invention porte également sur l'utilisation d'une tablette solide telle que définie ci-dessus, pour préparer une solution désinfectante de cuvette de toilettes WC, comprenant une concentration minimum efficace en chlore actif.

## Description des Figures

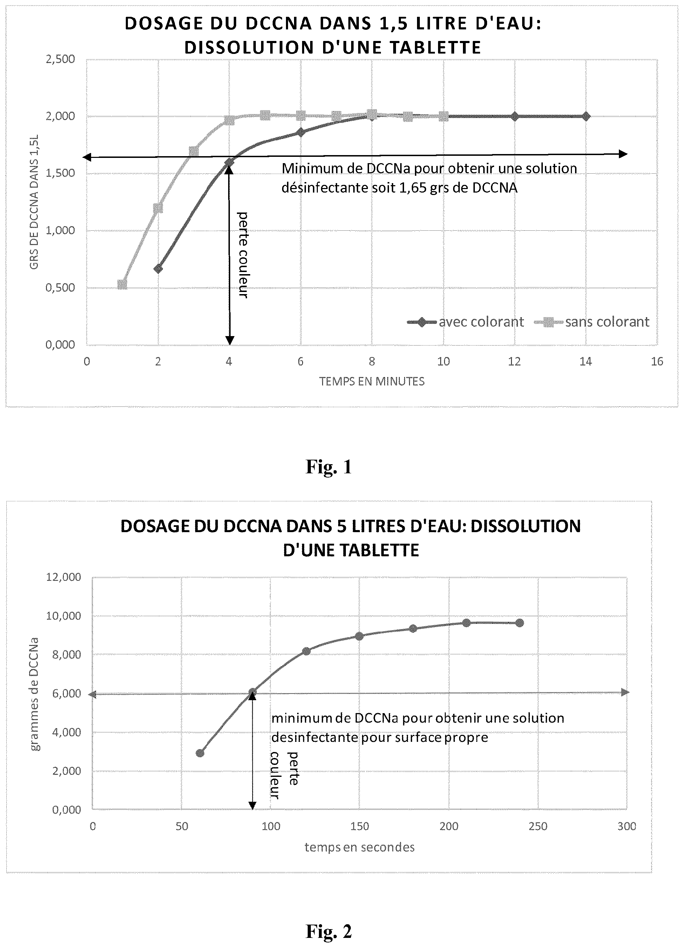

La Figure 1 représente le suivi de la concentration en chlore actif (exprimée en g/L) en fonction du temps (en minutes), après incorporation d'une tablette selon l'invention de 5 g dans une solution aqueuse de 1,5L et indique la variation de coloration. Ce suivi est également comparé au suivi de la concentration en chlore actif (exprimée en g/L) en fonction du temps (en minutes), après incorporation d'une tablette ne comprenant pas de colorant apte à colorer l'eau de 5 g dans une solution aqueuse de 1,5L.

La Figure 2 représente le suivi de la concentration en chlore actif (exprimée en g/L) en fonction du temps (en minutes), après incorporation d'une tablette selon l'invention de 20 g dans une solution aqueuse de 5L et indique la variation de coloration. Ce suivi est également comparé au suivi de la concentration en chlore actif (exprimée en g/L) en fonction du temps (en minutes), après incorporation d'une tablette ne comprenant pas de colorant apte à colorer l'eau de 20 g dans une solution aqueuse de 5L.

Les exemples qui suivent visent à illustrer la présente invention.

## Exemples

**Exemple 1 : Préparation de compositions solides selon l'invention**

Cet exemple décrit la composition et le procédé de préparation de 13 tablettes bicouches selon l'invention, de 1 tablette monocouche (essai 9) et de 1 poudre (essai 7).

Les tablettes testées ont une masse de 5 grammes, de forme cylindrique de diamètre 20mm. La composition de ces tablettes est résumée dans le tableau 1 suivant :

**Tableau 1 : Composition des tablettes testées**

| Essai 1 | Bicouche - ratio 100/0,1 - 1,5L | | |
|---|---|---|---|
| | **NOM** | % couche | % formule |
| **Couche 1** : 3g = couche active | DICHLOROISOCYANURATE DE SODIUM | 64,1 | 38,46 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |

(suite)

| Essai 1 | Bicouche - ratio 100/0,1 - 1,5L | | |
|---|---|---|---|
| **Couche 2 : 2g** = couche colorante | **NOM** | **% couche** | **% formule** |
| | BICARBONATE DE SODIUM | 59,3 | 23,72 |
| | ACIDE ADIPIQUE | 39,4 | 15,76 |
| | Croscarmellose | 1 | 0,4 |
| | PREMIX MENTHE CONIFERE | 0,2 | 0,08 |
| | BLUES NBL | 0,1 | 0,04 |
| Essai 2 | Bicouche - ratio 100/0,3 - 1,5L | | |
| **Couche 1 : 3g** = couche active | **NOM** | **% couche** | **% formule** |
| | DICHLOROISOCYANURATE DE SODIUM | 64,1 | 38,46 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |
| **Couche 2 : 2g** = couche colorante | **NOM** | **% couche** | **% formule** |
| | BICARBONATE DE SODIUM | 59,2 | 23,68 |
| | ACIDE ADIPIQUE | 39,3 | 15,72 |
| | Croscarmellose | 1 | 0,4 |
| | PREMIX MENTHE CONIFERE | 0,2 | 0,08 |
| | BLUES NBL | 0,3 | 0,12 |
| Essai 3 | Bicouche - ratio 100/1 - 1,5L | | |
| **Couche** 1 : **3g** = couche active | **NOM** | **% couche** | **% formule** |
| | DICHLOROISOCYANURATE DE SODIUM | 64,1 | 38,46 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |
| **Couche 2 : 2g** = couche colorante | **NOM** | **% couche** | **% formule** |
| | BICARBONATE DE SODIUM | 58,85 | 23,54 |
| | ACIDE ADIPIQUE | 38,97 | 15,59 |
| | Croscarmellose | 1 | 0,40 |
| | PREMIX MENTHE CONIFERE | 0,2 | 0,08 |
| | BLUES NBL | 0,98 | 0,39 |
| Essai 4 | Bicouche - ratio 100/2 - 1,5L | | |
| **Couche 1** : **3g** = couche active | **NOM** | **% couche** | **% formule** |
| | DICHLOROISOCYANURATE DE SODIUM | 64,1 | 38,46 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |

(suite)

| Essai 4 | Bicouche - ratio 100/2 - 1,5L | | |
|---|---|---|---|
| **Couche 2 : 2g** = couche colorante | **NOM** | **% couche** | **% formule** |
| | BICARBONATE DE SODIUM | 58,25 | 23,30 |
| | ACIDE ADIPIQUE | 38,59 | 15,44 |
| | Croscarmellose | 1 | 0,40 |
| | PREMIX MENTHE CONIFERE | 0,2 | 0,08 |
| | BLUES NBL | 1,96 | 0,78 |
| Essai 5 | Bicouche - ratio 100/0,35 - 1,5L | | |
| **Couche 1 : 2g** = couche colorante | **NOM** | **% couche** | **% formule** |
| | BICARBONATE DE SODIU | 59,15 | 23,66 |
| | ACIDE ADIPIQUE | 39,3 | 15,72 |
| | Croscarmellose | 1 | 0,40 |
| | PREMIX MENTHE CONIFERE | 0,2 | 0,08 |
| | BLUES NBL | 0,35 | 0,14 |
| **Couche** 2 : 3g = couche active | NOM | % couche | % for rmule |
| | DICHLOROISOCYANURATE DE SODIUM | 64,1 | 38,4 6 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |
| Essai 6 | Bicouche - ratio 100/1 - 1,5L | | |
| **Couche** 1 : **2g** = couche colorante | **NOM** | **% couche** | **% fo rmule** |
| | BICARBONATE DE SODIU | 58,85 | 23,5 4 |
| | ACIDE ADIPIQUE | 38,97 | 15,5 9 |
| | Croscarmellose | 1 | 0,40 |
| | PREMIX MENTHE CONIFERE | 0,2 | 0,08 |
| | BLUES NBL | 0,98 | 0,39 |
| **Couche 2 : 3g** = couche active | **NOM** | **% couche** | **% fo rmule** |
| | DICHLOROISOCYANURATE DE SODIU M | 64,1 | 38,4 6 |
| | BICARBONATE DE SODIUM | 20,92 | 12,5 5 |
| | ACIDE ADIPIQUE | 14,73 | 8,84 |
| | SODIUM LAURYL SULFOACETATE | 0,25 | 0,15 |
| Essai 7 (comparaif) | Poudre - ratio 100/0,3 - 1,5L | | |
| **Poudre 1** : **3g** = poudre active | **NOM** | **% poudre 1** | **% for rmule** |
| | DICHLOROISOCYANURATE DE SODIUM | 64,1 | 38,4 6 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |

(suite)

| Essai 7 (comparaif) | Poudre - ratio 100/0,3 - 1,5L | | |
|---|---|---|---|
| **Poudre 2 : 2g** = poudre colorante | NOM | % poudre 2 | % fo rmule |
| | BICARBONATE DE SODIUM | 59,2 | 23,6 8 |
| | ACIDE ADIPIQUE | 39,3 | 15,7; 2 |
| | Croscarmellose | 1 | 0,4 |
| | PREMIX MENTHE CONIFERE | 0,2 | 0,08 |
| | BLUES NBL | 0,3 | 0,12 |
| Essai 8 | Bicouche - ratio 100/10 - 1,5L | | |
| **Couche 1 : 3g** = couche active | NOM | % couche | % fo rmule |
| | DICHLOROISOCYANURATE DE SODIUM | 64,1 | 38,4 6 |
| | BICARBONATE DE SODIUM | 20,92 | 12,55 |
| | ACIDE ADIPIQUE | 14,73 | 8,84 |
| | SODIUM LAURYL SULFOACETATE | 0,25 | 0,15 |
| **Couche 2 : 2g** = couche colorante | NOM | % couche | % fo rmule |
| | BICARBONATE DE SODIUM | 53,48 | 21,39 |
| | ACIDE ADIPIQUE | 35,52 | 14,21 |
| | Croscarmellose | 1 | 0,40 |
| | PREMIX MENTHE CONIFERE | 0,2 | 0,08 |
| | BLUES NBL | 9,8 | 3,92 |
| Essai 9 (comparatif) | Monocouche - ratio 100/1 - 1,5L | | |
| **Couche 1 : 5g Couche 1: 5g** = couche active + colorante | NOM | % couche | % formule |
| | DICHLOROISOCYANURATE DE SODI UM | - | 38,46 |
| | BICARBONATE DE SODIUM | - | 36,21 |
| | ACIDE ADIPIQUE | - | 24,55 |
| | SODIUM LAURYL SULFOACETATE | - | 0,15 |
| | Croscarmellose | - | 0,4 |
| | PREMIX MENTHE CONIFERE | - | 0,08 |
| | BLUES NBL | - | 0,15 |
| **Essa: 10** | Bicouche - ratio 100/0,1 - 5L | | |
| **Couche 1 : 15g** = couche active | NOM | % couche | % formule |
| | DICHLOROISOCYANURATE DE SODI UM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,25 | 0,19 |

(suite)

| **Essa: 10** | **Bicouche - ratio 100/0,1 - 5L** | | |
|---|---|---|---|
| **Couche 2 : 5g** = couche colorante | **NOM** | **% couche** | **% formule** |
| | BICARBONATE DE SODIUM | 59,2 | 14,8 |
| | ACIDE ADIPIQUE | 39,4 | 9,85 |
| | Croscarmellose | 1 | 0,25 |
| | PREMIX MENTHE CONIFERE | 0,2 | 0,05 |
| | BLUES NBL | 0,2 | 0,05 |
| **Essai 11** | **Bicouche - ratio 100/0,26 - 5L** | | |
| **Couche 1 : 15g** = couche active | **NOM** | **% couche** | **% formule** |
| | DICHLOROISOCYANURATE DE SODIUM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,25 | 0,19 |
| **Couche 2 : 5g** = couche colorante | **NOM** | **% couche** | **% formule** |
| | BICARBONATE DE SODIUM | 59,13 | 14,7825 |
| | ACIDE ADIPIQUE | 39,15 | 9,7875 |
| | Croscarmellose | 1 | 0,25 |
| | PREMIX MENTHE CONIFERE | 0,2 | 0,05 |
| | BLUES NBL | 0,52 | 0,13 |
| **Essai 12** | **Bicouche - ratio 100/1 - 5L** | | |
| **Couche 1 : 15g** = couche active | **NOM** | **% couche** | **% formule** |
| | DICHLOROISOCYANURATE DE SODIUM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,25 | 0,19 |
| **Couche 2 : 5g** = couche colorante | **NOM** | **% couche** | **% formule** |
| | BICARBONATE DE SODIUM | 58,3 | 14,575 |
| | ACIDE ADIPIQUE | 38,5 | 9,625 |
| | Croscarmellose | 1 | 0,25 |
| | PREMIX MENTHE CONIFERE | 0,2 | 0,05 |
| | BLUES NBL | 2 | 0,5 |
| **Essai 13** | **Bicouche - ratio 100/2 - 5L** | | |
| **Couche 1 : 15g** = couche active | **NOM** | **% couche** | **% formule** |
| | DICHLOROISOCYANURATE DE SODIUM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,25 | 0,19 |

(suite)

| Essai 13 | Bicouche - ratio 100/2 - 5L | | |
|---|---|---|---|
| **Couche 2** : **5g** = couche colorante | **NOM** | **% couche** | **% formule** |
| | BICARBONATE DE SODIUM | 57,3 | 14,325 |
| | ACIDE ADIPIQUE | 37,5 | 9,375 |
| | Croscarmellose | 1 | 0,25 |
| | PREMIX MENTHE CONIFERE | 0,2 | 0,05 |
| | BLUES NBL | 4 | 1 |
| Essai 14 | Bicouche - ratio 100/0,3 - 5L | | |
| **Couche 1** : **5g** = couche colorante | **NOM** | **% couche** | **% formule** |
| | BICARBONATE DE SODIUM | 59,3 | 14,825 |
| | ACIDE ADIPIQUE | 39,4 | 9,85 |
| | Croscarmellose | 1 | 0,25 |
| | PREMIX MENTHE CONIFERE | 0,2 | 0,05 |
| | BLUES NBL | 0,1 | 0,025 |
| **Couche 2 : 15g** = couche active | **NOM** | **% couche** | **% formule** |
| | DICHLOROISOCYANURATE DE SODIUM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,2 | 0,15 |
| Essai 15 | Bicouche - ratio 100/1 - 5L | | |
| **Couche 1** : **5g** | **NOM** | **% couche** | **% formule** |
| = couche colorante | BICARBONATE DE SODIUM | 58,3 | 14,575 |
| | ACIDE ADIPIQUE | 38,5 | 9,625 |
| | Croscarmellose | 1 | 0,25 |
| | PREMIX MENTHE CONIFERE | 0,2 | 0,05 |
| | BLUES NBL | 2 | 0,5 |
| **Couche 2 : 15g** = couche active | **NOM** | **% couche** | **% formule** |
| | DICHLOROISOCYANURATE DE SODIUM | 64,1 | 48,08 |
| | BICARBONATE DE SODIUM | 20,92 | 15,69 |
| | ACIDE ADIPIQUE | 14,73 | 11,05 |
| | SODIUM LAURYL SULFOACETATE | 0,25 | 0,19 |
| *Les pourcentages sont exprimés en masse par rapport à la masse totale de la composition* | | | |

La tablette monocouche est produite par mélange de l'ensemble des matières premières sous forme de poudre puis par compression sur une presse rotative avec une force de compression de 50 kN.

La poudre est produite par simple mélange des matières premières sous forme de poudre. Les tablettes bicouches sont produites selon le procédé suivant :

a) Mélange des différents composants de la couche 1 de façon homogène ;
b) Pré-compaction de la composition 1 par compression directe sur une presse rotative avec une force de compression de 10 kN ;
c) Mélange des différents composants de la couche 2 de façon homogène ;

d) Pré-compaction de la composition 2 sur la couche 1 préalablement formée à l'étape b), par compression directe sur une presse rotative avec une force de compression de 10 kN ;

e) Compaction de l'ensemble des couches obtenue après l'étape d), par compression directe sur une presse rotative avec une force de compression de 40 kN, pour obtenir une tablette solide.

**Exemple 2 : Test d'efficacité des compositions selon l'invention**

Afin de mesurer l'efficacité des compositions selon l'invention, les tests suivants sont effectués :

- Observation de la coloration initiale de la solution désinfectante ;
- Observation du délitement de la tablette ;
- Mesure de la concentration en chlore actif au changement de couleur de la solution désinfectante ; et
- Test de vieillissement.

Ces tests sont effectués sur les 13 tablettes bicouches citées dans l'exemple 1. Les résultats sont ensuite comparés avec les résultats obtenus pour la poudre (essai 7) et la tablette monocouche (essai 9), également citées dans l'exemple 1.

> *Condition pour l'observation de la coloration initiale de la solution désinfectante :*

Pour les essais 1 à 9, la tablette (ou poudre) est plongée dans 1,5 L d'eau et la coloration initiale de l'eau apparait dès les premières secondes de dissolution de la tablette.

Pour les essais 10 à 15, la tablette est plongée dans 5 L d'eau et la coloration initiale de l'eau apparait dès les premières secondes de dissolution de la tablette.

> *Condition pour l'observation du délitement de la tablette :*

Pour les essais 1 à 9, la tablette (ou poudre) est plongée dans 1,5 L d'eau et le délitement de la tablette est observé.

Pour les essais 10 à 15, la tablette est plongée dans 5 L d'eau et le délitement de la tablette est observé

➢ *Condition pour la mesure de la concentration en chlore actif au changement de couleur de la solution désinfectante* :

La tablette (ou poudre) est plongée dans 1,5 L d'eau (pour les essais 1 à 9) ou 5 L d'eau (pour les essais 10 à 15), et la coloration de la solution désinfectante est observée. De l'introduction de la tablette dans la solution aqueuse jusqu'à sa dissolution complète, la concentration en chlore actif est mesurée selon le protocole suivant :

*Principe*

La teneur en chlore actif est dosée par iodométrie.

*Appareillage*

- ✔ Burette de 25 ou 50 ml
- ✔ 2 erlens de 250 ml à col large
- ✔ Balance de laboratoire avec une précision de $\pm$ 0,01 g
- ✔ Thiosulfate de sodium (Na2S2O3) de concentration 0,1 N
- ✔ Solution à 10 % de KI
- ✔ $H_2SO_4$ 4N
- ✔ Solution d'empois d'amidon à 5 g/L
- ✔ Eprouvette de 25 mL

*Mode opératoire*

✔ Introduire dans l'erlen la prise d'essai de masse m
✔ Ajouter successivement :

- 100 ml d'eau distillée

- 10 ml de solution de KI 10%

- 15 ml d'acide sulfurique (H2SO4 4 N)

✔ Couler la solution de thiosulfate de sodium (Na2S2O3) sous agitation
✔ Lorsque la solution devient jaune paille, ajouter environ 1 ml d'empois d'amidon : la solution devient alors bleu - gris.
✔ Continuer à couler la solution de thiosulfate de sodium au goutte à goutte jusqu'à la décoloration complète. Noter le volume coulé V.

*Résultat*

Chlore disponible (sous forme de Cl), $\% = \dfrac{3{,}545 \times N \times V}{m}$   Où :

N : normalité de la solution de thiosulfate de sodium (0,1N)
V : volume coulé (mL)
m : masse de la prise d'essai en g.

> *Condition pour le test de vieillissement :*

La tablette (ou poudre) est plongée dans 1,5 L d'eau (pour les essais 1 à 9) ou 5 L d'eau (pour les essais 10 à 15), et le test de vieillissement est effectué selon le protocole suivant :

*Principe*

Etudier l'évolution de colorations, masses, duretés, aspects des pastilles mises en étuve, et la variation de la composition chimique (chlore actif, oxygène actif, alcalinité) suivant la nature des produits étudiés.

*Appareillage*

✔ 1 étuve à 40° C
✔ Enceinte climatique Vötsch
✔ 1 balance
✔ 1 appareil de dureté
✔ 1 pied à coulisse n° IMMO 025
✔ test de dissolution statique n° IO.DIS.1

*Mode opératoire*

Les tablettes et poudre citées à l'exemple 1 sont introduites dans une étuve à 40 °C et en enceinte climatique (en parallèle à température ambiante), pendant 1 mois. Un point hebdomadaire est réalisé.
Au début et à la fin de l'étude, les tests suivants sont effectués et les résultats comparés :

- mesures de masse

- mesures de dureté

- dissolution

- aspect visuel des pastilles

- coloration

- contrôle du chlore actif

> *Résultats*

Les résultats des tests cités ci-dessus sont regroupés dans le Tableau 2 suivant. Pour rappel, les tablettes des essais 1 à 9 sont plongées dans 1,5 L d'eau et les tablettes des essais 10 à 15 sont plongées dans 5 L d'eau.

**Tableau 2 : présentation des résultats**

| Essai | Ratio DCCNa/colorant | Coloration de la solution initiale | Délitement | Disparition de la couleur dans le temps en solution | Stabilité - test vieillissement |
|---|---|---|---|---|---|
| 1 | *100 / 0,1* | Faible intensité mais détectable par le consommateur | Délitement de la couche colorée débutant quelques secondes avant celui de la couche chlorée | Après avoir libéré 1,50grs de chlore | OK 1 mois EQUIVALENT 2 ans |
| 2 | 100/0,3 | Bonne intensité de la couleur | IDEM essai 1 | Après avoir libéré 1,65grs de chlore | OK 1 mois EQUIVALENT 2 ans |
| 3 | 100 / 1 | Forte intensité | IDEM essai 1 | Après avoir libéré 1,65grs de chlore mais faible coloration jaunâtre restante | OK 1 mois EQUIVALENT 2 ans |
| 4 | 100/2 | Très forte intensité | IDEM essai 1 | Après avoir libéré 1,65grs de chlore mais coloration jaunâtre intense très persitante | OK 1 mois EQUIVALENT 2 ans |
| 5 | 100/0,35 | Très Faible intensité | Début du délitement de la couche chlorée avant celui de la couche coloré | Après avoir libéré 1,65grs de chlore | OK 1 mois EQUIVALENT 2 ans |
| 6 | 100/1 | Faible intensité | IDEM essai 5 | Après avoir libéré 1,65grs de chlore mais faible coloration jaunâtre restante | OK 1 mois EQUIVALENT 2 ans |
| 7 Poudre | 100/0,3 | Coloration faible qui disparaît tres rapidement | Tout en même temps | Disparition très rapide | Aucune stabilité colorant pas stable |
| 8 | 100/10 | Très forte intensité | IDEM essai 1 | Après avoir libéré 1,65grs de chlore mais coloration jaunâtre très intense et très très persitante | OK 1mois EQUIVALENT 2 ans |

(suite)

| Essai | Ratio DCCNa/ colorant | Coloration de la solution initiale | Délitement | Disparition de la couleur dans le temps en solution | Stabilité - test vieillissement |
|---|---|---|---|---|---|
| **9** **Monocouche** | 100/1 | Coloration faible mais qui disparaît très vite | Tout en même temps | Difficile de voir le virage | Problème de stabilité apres 1 semaines (équivalence à 6 mois maximum). Plus de coloration |
| | | | | | |
| **10** | *100 / 0,1* | Faible intensité mais détectable par le consommateur | Délitement de la couche colorée débutant quelques secondes avant celui de la couche chlorée | Après avoir libéré 6 grs de chlore | OK 1 mois EQUIVALENT 2 ans |
| **11** | 100 / 0,26 | Bonne intensité de la couleur | IDEM essai 1 | Après avoir libéré 6 grs de chlore | OK 1 mois EQUIVALENT 2 ans |
| **12** | 100 / 1 | Forte intensité | IDEM essai 1 | Après avoir libéré 5,8 grs de chlore mais faible coloration jaunâtre restante | OK 1 mois EQUIVALENT 2 ans |
| **13** | 100/2 | Très forte intensité | IDEM essai 1 | Après avoir libéré 5,8grs de chlore mais coloration jaunâtre intense très persitante | OK 1 mois EQUIVALENT 2 ans |
| **14** | 100/0,3 | Très Faible intensité | Début du délitement de la couche chlorée avant celui de la couche coloré | Après avoir libéré 5,6 grs de chlore | OK 1 mois EQUIVALENT 2 ans |
| **15** | 100/1 | Faible intensité | Début du délitement de la couche chlorée avant celui de la couche coloré | Après avoir libéré 5,9grs de chlore mais faible coloration jaunâtre restante | OK 1 mois EQUIVALENT 2 ans |

La tablette bicouche Eurotab comprenant le colorant bleu outre-mer 24 colorant la poudre de la couche ne comprenant pas de DCCNa en bleu dans un ratio DCCNa/colorant de 100/0,04 a également été testée. Après introduction dans 1,5 L d'eau, aucune coloration n'est observée. Ce dernier test montre la nécessité d'utiliser un colorant apte à colorer l'eau.

**Exemple 3 : Analyse de la perte de coloration de la solution désinfectante en fonction de la concentration en chlore actif libéré**

Le dosage du chlore actif en fonction du temps a été effectué pour les solutions désinfectantes des essais 1 à 6 (1,5L) et 10 à 15 (5L). La concentration en chlore actif dans la solution désinfectante est mesurée en fonction du temps écoulé après incorporation de la tablette dans la l'eau. La disparition ou la variation de la coloration est observée en parallèle.

Le protocole de dosage du chlore actif est identique à celui exposé dans l'exemple 2.

Les Figures 1 et 2 représentent le suivi moyen de la concentration en chlore actif (exprimée en g/L) dans la solution désinfectante en fonction du temps (en minutes). Ces Figures mettent en évidence que la coloration disparait au moment où la concentration en DCCNa d'eau atteint 1,65 grs dans 1,5L et 5,4grs dans 5L d'eau.

La dose minimale de DCCNa pour que la solution soit considérée comme désinfectante est de 1,53 grs de DCCNa pour 1,5l d'eau selon la norme NF EN 1276 (désinfection bactéricide en suspension condition saleté - mars 2010).

La dose minimale pour que la solution soit considérée comme désinfectante est de 1,61 grs de DCCNa pour 1,5L d'eau selon la norme NF EN 13697 (désinfection bactéricide de surface en condition de propreté - juin 2015) et de 5,2 grs de DCCNa pour 5L d'eau selon la norme NF EN 13697 (bactéricide de surface condition de propreté pour désinfecter un plan de travail un sol - juin 2015).

Ainsi, ce test démontre que lorsque la coloration disparait ou varie, la solution obtenue est bien une solution désinfectante selon les 2 normes précitées. Le virage de coloration est obtenu au moment où la solution devient une solution désinfectante.

Les Figures 1 et 2 comparent également le suivi de la concentration en chlore actif dans la solution désinfectante en fonction du temps pour les tablettes selon l'invention et pour des tablettes comparatives identiques aux tablettes testées mais dans lesquelles le colorant a été remplacé par de l'agent effervescent (bicarbonate de sodium/acide adipique).

**Revendications**

1. Procédé de préparation d'une solution désinfectante comprenant les étapes suivantes :

    - Placer une tablette solide désinfectante dans une solution aqueuse, avantageusement l'eau ; et
    - Attendre la disparition ou le changement de coloration de ladite solution aqueuse, indiquant que la concentration minimale efficace en chlore actif est atteinte,

    **caractérisé en ce que** la tablette solide désinfectante comprend au moins une couche active comprenant un agent chloré en quantité suffisante pour obtenir une solution aqueuse désinfectante, et au moins une couche colorante comprenant un colorant apte à colorer la solution aqueuse et ne comprenant pas d'agent chloré, le ratio en poids agent chloré / colorant étant d'au moins 100/0,005, avantageusement compris de 100/0,005 à 100/10.

2. Procédé selon la revendication 1, dans lequel lorsque la tablette solide est placée dans la solution aqueuse, la couche colorante de la tablette solide désinfectante commence à se déliter avant la couche active.

3. Procédé selon la revendication 1 ou 2, dans lequel l'agent chloré est choisi parmi les dichloroisocyanurates, avantageusement l'agent chloré est le dichloroisocyanurate de sodium, le dichloroisocyanurate de potassium, ou leur mélange.

4. Procédé selon l'une quelconque des revendications 1 à 3, dans lequel le poids de la couche active est compris de 0,1 g à 30 g, avantageusement de 0,5 g à 30 g, avantageusement de 1 g à 25 g, plus avantageusement de 3 g à 15 g, et le poids de la couche colorante est compris de 0,1 g à 20 g, avantageusement 0,3 g à 20 g, avantageusement de 0,3 g à 8 g, plus avantageusement de 2 g à 5 g.

5. Procédé selon l'une quelconque des revendications 1 à 4, dans lequel la couche active comprend en outre au moins un agent permettant d'agir sur la dissolution de la composition, tel qu'un agent effervescent, un agent de désintégration, un agent d'éclatement ou leurs mélanges.

6. Procédé selon la revendication 5, dans lequel la couche active comprend de 3 % à 98 % d'agent chloré et de 1 % à 95 % d'agent permettant d'agir sur la dissolution de la composition, tel qu'un agent effervescent, un agent de désintégration, un agent d'éclatement ou leurs mélanges.

7. Procédé selon l'une quelconque des revendications 1 à 6, dans lequel la couche colorante comprend en outre au moins un agent permettant d'agir sur la dissolution de la composition, tel qu'un agent effervescent, un agent de désintégration, un agent d'éclatement ou leurs mélanges.

8. Procédé selon la revendication 7, dans lequel la couche colorante comprend de 0,005 % à 10 % de colorant et de 0,1 % à 99,995 % d'agent permettant d'agir sur la dissolution de la composition, tel qu'un agent effervescent, un agent de désintégration, un agent d'éclatement ou leurs mélanges.

**9.** Tablette solide désinfectante **caractérisée en ce que** la tablette solide désinfectante comprend au moins une couche active comprenant un agent chloré en quantité suffisante pour obtenir une solution aqueuse désinfectante, et au moins une couche colorante comprenant un colorant apte à colorer la solution aqueuse et ne comprenant pas d'agent chloré,

le ratio en poids agent chloré / colorant étant d'au moins 100/0,005, avantageusement compris de 100/0,005 à 100/10.

**10.** Tablette selon la revendication 9, dans laquelle l'agent chloré est choisi parmi les dichloroisocyanurates, avantageusement l'agent chloré est le dichloroisocyanurate de sodium, le dichloroisocyanurate de potassium, ou leur mélange.

**11.** Tablette selon la revendication 9 ou 10, dans laquelle le poids de la couche active est compris de 0,1 g à 30 g, avantageusement de 0,5 g à 30 g, avantageusement de 1 g à 25 g, plus avantageusement de 3 g à 15 g, et le poids de la couche colorante est compris de 0,1 g à 20 g, avantageusement 0,3 g à 20 g, avantageusement de 0,3 g à 8 g, plus avantageusement de 2 g à 5 g.

**12.** Tablette selon l'une quelconque des revendications 9 à 11, dans laquelle la couche active comprend en outre au moins un agent permettant d'agir sur la dissolution de la composition, tel qu'un agent effervescent, un agent de désintégration, un agent d'éclatement ou leurs mélanges.

**13.** Tablette selon la revendication 12, dans laquelle la couche active comprend de 3 % à 98 % d'agent chloré et de 1 % à 95 % d'agent permettant d'agir sur la dissolution de la composition, tel qu'un agent effervescent, un agent de désintégration, un agent d'éclatement ou leurs mélanges.

**14.** Tablette selon l'une quelconque des revendications 9 à 13, dans laquelle la couche colorante comprend en outre au moins un agent permettant d'agir sur la dissolution de la composition, tel qu'un agent effervescent, un agent de désintégration, un agent d'éclatement ou leurs mélanges.

**15.** Tablette selon la revendication 14, dans laquelle la couche colorante comprend de 0,005 % à 10 % de colorant et de 0,1 % à 99,995 % d'agent permettant d'agir sur la dissolution de la composition, tel qu'un agent effervescent, un agent de désintégration, un agent d'éclatement ou leurs mélanges.

**16.** Procédé de préparation d'une tablette solide telle que définie dans l'une quelconque des revendications 9 à 15, comprenant les étapes suivantes :

f) mélanger les différents composants de la couche active de façon homogène, dont au moins un agent chloré en quantité suffisante pour libérer 1,02 g/L de chlore actif dans une solution aqueuse , pour former la composition active;

g) pré-compacter la composition active, en particulier par compression directe, par exemple sur des presses rotatives ;

h) mélanger les différents composants de la couche colorante de façon homogène, dont au moins un colorant apte à colorer une solution aqueuse , pour former la composition colorante;

i) pré-compacter la composition colorante sur la couche active préalablement formée à l'étape b), en particulier par compression directe, par exemple sur des presses rotatives ;

j) éventuellement renouveler les étapes a) à d) autant de fois que de couches nécessaires ; et

k) compacter l'ensemble des couches obtenue après l'étape d) ou e), en particulier par compression directe, par exemple sur des presses rotatives, pour obtenir une tablette solide,

l'ordre des étapes a)-b) et c)-d) pouvant éventuellement être inversé.

**17.** Utilisation d'une tablette solide telle que définie selon l'une quelconque des revendications 9 à 15, pour préparer une solution désinfectante comprenant une concentration minimum efficace en chlore actif.

**Patentansprüche**

**1.** Zubereitungsverfahren einer desinfizierenden Lösung, umfassend die folgenden Schritte:

- eine desinfizierende, feste Tablette in eine wässerige Lösung, vorteilhafterweise Wasser, legen; und

- das Verschwinden oder die Farbänderung der genannten wässerigen Lösung abwarten, die anzeigt, dass die wirksame Mindestkonzentration an aktivem Chlor erreicht ist,

**dadurch gekennzeichnet, dass** die desinfizierende feste Tablette wenigstens eine aktive Schicht umfasst, umfassend einen Wirkstoff, der mit einer ausreichenden Menge gechlort wurde, um eine desinfizierende wässerige Lösung zu erhalten, und wenigstens eine färbende Schicht, umfassend einen Farbstoff, der geeignet ist, um die wässerige Lösung zu färben, und nicht umfassend einen gechlorten Wirkstoff,
wobei das Gewichtsverhältnis gechlorter Wirkstoff / Farbstoff wenigstens 100/0,005 beträgt, vorteilhafterweise zwischen 100/0,005 und 100/10 inbegriffen ist.

2. Verfahren gemäß Anspruch 1, bei dem, wenn die feste Tablette in die wässerige Lösung gelegt wird, die färbende Schicht der desinfizierenden festen Tablette beginnt, sich vor der aktiven Schicht aufzulösen.

3. Verfahren gemäß Anspruch 1 oder 2, bei dem der gechlorte Wirkstoff aus Dichlorisocyanuraten ausgewählt wird, der gechlorte Wirkstoff vorteilhafterweise Natrium-Dichlorisocyanurat, Kalium-Dichlorisocyanurat oder deren Mischung ist.

4. Verfahren gemäß irgendeinem der Ansprüche 1 bis 3, bei dem das Gewicht der aktiven Schicht zwischen 0,1 g und 30 g, vorteilhafterweise zwischen 0,5 g und 30 g, vorteilhafterweise zwischen 1 g und 25 g, noch vorteilhafterweise zwischen 3 g und 15 g inbegriffen ist und das Gewicht der färbenden Schicht zwischen 0,1 g und 20 g, vorteilhafterweise zwischen 0,3 g und 20 g, vorteilhafterweise zwischen 0,3 g und 20 g, vorteilhafterweise zwischen 0,3 g und 8 g, noch vorteilhafterweise zwischen 2 g und 5 g inbegriffen ist.

5. Verfahren gemäß irgendeinem der Ansprüche 1 bis 4, bei dem die aktive Schicht darüber hinaus wenigstens einen Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

6. Verfahren gemäß Anspruch 5, bei dem die aktive Schicht zwischen 3 % und 98 % gechlorten Wirkstoff und zwischen 1 % und 95 % Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

7. Verfahren gemäß irgendeinem der Ansprüche 1 bis 6, bei dem die färbende Schicht darüber hinaus wenigstens einen Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

8. Verfahren gemäß Anspruch 7, bei dem die färbende Schicht 0,005 % bis 10 % Färbemittel und 0,1 % bis 99,995 % Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

9. Desinfizierende, feste Tablette, **dadurch gekennzeichnet, dass** die desinfizierende, feste Tablette wenigstens eine aktive Schicht umfasst, umfassend einen Wirkstoff, der mit einer ausreichenden Menge gechlort ist, um eine desinfizierende wässerige Lösung zu erhalten, und wenigstens eine färbende Schicht, umfassend einen Farbstoff, der geeignet ist, um die wässerige Lösung zu färben und keinen gechlorten Wirkstoff umfasst,
wobei das Verhältnis gechlorter Wirkstoff / Farbstoff wenigstens 100/0,005 beträgt, vorteilhafterweise zwischen 100/0,005 und 100/10 inbegriffen ist.

10. Tablette gemäß Anspruch 9, bei der der gechlorte Wirkstoff aus Dichlorisocyanuraten ausgewählt ist, der gechlorte Wirkstoff vorteilhafterweise Natrium-Dichlorisocyanurat, Kalium-Dichlorisocyanurat oder deren Mischung ist.

11. Tablette gemäß Anspruch 9 oder 10, bei der das Gewicht der aktiven Schicht zwischen 0,1 g und 30 g, vorteilhafterweise zwischen 0,5 g und 30 g, vorteilhafterweise zwischen 1 g und 25 g, noch vorteilhafterweise zwischen 3 g und 15 g inbegriffen ist und das Gewicht der färbenden Schicht zwischen 0,1 g und 20 g, vorteilhafterweise 0,3 g und 20 g, vorteilhafterweise zwischen 0,3 g und 8 g, noch vorteilhafterweise zwischen 2 g und 5 g inbegriffen ist.

12. Tablette gemäß irgendeinem der Ansprüche 9 bis 11, bei der die aktive Schicht darüber hinaus wenigstens einen Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

13. Tablette gemäß Anspruch 12, bei der die aktive Schicht 3 % bis 98 % gechlorten Wirkstoff und 1 % bis 95 % Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

14. Tablette gemäß irgendeinem der Ansprüche 9 bis 13, bei det die färbende Schicht darüber hinaus wenigstens einen Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

15. Tablette gemäß Anspruch 14, bei der die färbende Schicht 0,005 % bis 10 % Färbemittel und 0,1 % bis 99,995 % Wirkstoff umfasst, der das Einwirken auf die Auflösung der Zusammensetzung zulässt, wie z. B. einen aufschäumenden Wirkstoff, einen Auflösungswirkstoff, einen Zersplitterungswirkstoff oder deren Mischungen.

16. Zubereitungsverfahren einer festen Tablette gemäß Definition in irgendeinem der Ansprüche 9 bis 15, umfassend die folgenden Schritte:

   f) Mischen der unterschiedlichen Bestandteile der aktiven Schicht auf homogene Weise, darunter wenigstens einen gechlorten Wirkstoff in ausreichender Menge, um 1,02 g/l aktives Chlor in einer wässerigen Lösung freizusetzen, um die aktive Zusammensetzung zu bilden;
   g) Vorkompaktieren der aktiven Zusammensetzung, insbesondere per direktem Komprimieren, zum Beispiel auf Rotationspressen;
   h) Mischen der unterschiedlichen Bestandteile der färbenden Schicht auf homogene Weise, darunter wenigstens einen Farbstoff, der zum Färben einer wässerigen Lösung geeignet ist, um die färbende Zusammensetzung zu bilden;
   i) Vorkompaktieren der färbenden Zusammensetzung auf der zuvor in Schritt b) gebildeten aktiven Schicht, insbesondere durch direktes Komprimieren, zum Beispiel auf Rotationspressen;
   j) Eventuelles Wiederholen der Schritte a) bis d) so häufig, wie Schichten notwendig sind; und
   k) Kompaktieren aller nach Schritt d) oder e) erhaltenen Schichten, insbesondere per direktem Komprimieren, zum Beispiel auf Rotationspressen, um eine feste Tablette zu erhalten,

   wobei die Reihenfolge der Schritte a) - b) und c) - d) eventuell umgekehrt sein kann.

17. Verwendung einer festen Tablette gemäß Definition gemäß irgendeinem der Ansprüche 9 bis 15 zum Zubereiten einer desinfizierenden Lösung, umfassend eine wirksame Mindestkonzentration an aktivem Chlor.

## Claims

1. A method for preparing a disinfectant solution comprising the following steps:

   - Placing a solid disinfectant tablet in an aqueous solution, preferably water; and
   - Waiting for the disappearance or the change in coloration of said aqueous solution, indicating that the minimum effective concentration of active chlorine has been reached,

   **characterized in that** the solid disinfectant tablet comprises at least one active layer comprising a chlorinated agent in a sufficient amount to obtain an aqueous disinfectant solution, and at least one coloring layer comprising a colorant capable of coloring the aqueous solution and not comprising a chlorinated agent, the chlorinated agent/colorant weight ratio being at least 100/0.005, advantageously of between 100/0.005 and 100/10.

2. The method according to claim 1, wherein when the solid tablet is placed in the aqueous solution, the coloring layer of the solid disinfectant tablet begins to disintegrate before the active layer.

3. The method according to claim 1 or 2, wherein the chlorinated agent is selected from dichloroisocyanurates, advantageously the chlorinated agent is sodium dichloroisocyanurate, potassium dichloroisocyanurate, or a mixture thereof.

4. The method according to any of claims 1 to 3, wherein the weight of the active layer is between 0.1 g and 30 g, advantageously between 0.5 g and 30 g, advantageously between 1 g and 25 g, more advantageously between 3 g and 15 g, and the weight of the coloring layer is of between 0.1 g and 20 g, advantageously between 0.3 g and 20 g, advantageously between 0.3 g and 8 g, more advantageously between 2 g and 5 g.

5. The method according to any of claims 1 to 4, wherein the active layer further comprises at least one agent for affecting the dissolution of the composition, such as an effervescent agent, disintegrating agent, bursting agent or mixtures thereof.

6. The method according to claim 5, wherein the active layer comprises from 3% to 98% of a chlorinated agent and from 1% to 95% of an agent for affecting the dissolution of the composition, such as an effervescent agent, disintegrating agent, bursting agent, or mixtures thereof.

7. The method according to any of claims 1 to 6, wherein the coloring layer further comprises at least one agent for affecting the dissolution of the composition, such as an effervescent agent, disintegrating agent, bursting agent, or mixtures thereof.

8. The method according to claim 7, wherein the coloring layer comprises from 0.005% to 10% of a colorant and from 0.1% to 99.995% of an agent for affecting the dissolution of the composition, such as an effervescent agent, disintegrating agent, bursting agent, or mixtures thereof.

9. A disinfectant solid tablet, **characterized in that** the disinfectant solid tablet comprises at least one active layer comprising a chlorinated agent in a sufficient amount to obtain a disinfectant aqueous solution, and at least one coloring layer comprising a colorant capable of coloring the aqueous solution and not comprising a chlorinated agent, the chlorinated agent/colorant weight ratio being at least 100/0.005, advantageously of between 100/0.005 and 100/10.

10. The tablet according to claim 9, wherein the chlorinated agent is selected from dichloroisocyanurates, advantageously the chlorinated agent is sodium dichloroisocyanurate, potassium dichloroisocyanurate, or a mixture thereof.

11. The tablet according to claim 9 or 10, wherein the weight of the active layer is between 0.1 g and 30 g, advantageously between 0.5 g and 30 g, advantageously between 1 g and 25 g, more advantageously between 3 g and 15 g, and the weight of the coloring layer is between 0.1 g to 20 g, advantageously between 0.3 g and 20 g, advantageously from 0.3 g and 8 g, more advantageously between 2 g and 5 g.

12. The tablet according to any of claims 9 to 11, wherein the active layer further comprises at least one agent for affecting the dissolution of the composition, such as an effervescent agent, disintegrating agent, bursting agent or mixtures thereof.

13. The tablet according to claim 12, wherein the active layer comprises from 3% to 98% of a chlorinated agent and from 1% to 95% of an agent for affecting the dissolution of the composition, such as an effervescent agent, disintegrating agent, bursting agent, or mixtures thereof.

14. The tablet according to any of claims 9 to 13, wherein the coloring layer further comprises at least one agent for affecting the dissolution of the composition, such as an effervescent agent, disintegrating agent, bursting agent, or mixtures thereof.

15. The tablet according to claim 14, wherein the coloring layer comprises from 0.005% to 10% of a colorant and from 0.1% to 99.995% of an agent for affecting the dissolution of the composition, such as an effervescent agent, disintegrating agent, bursting agent, or mixtures thereof.

16. A method for preparing a solid tablet as defined in any of claims 9 to 15, comprising the following steps of:

f) homogeneously mixing the various components of the active layer, including at least one chlorinated agent in a sufficient amount to release 1.02 g/L of active chlorine into an aqueous solution, to form the active composition;
g) pre-compacting the active composition, in particular by direct compression, for example on rotary presses;
h) homogeneously mixing the various components of the coloring layer, including at least one colorant capable of coloring an aqueous solution, to form the coloring composition;
i) pre-compacting the coloring composition on the active layer previously formed in step b), in particular by direct compression, for example on rotary presses;

j) optionally repeating steps a) to d) as many times as the number of layers required; and

k) compacting all the layers obtained after step d) or e), in particular by direct compression, for example on rotary presses, to obtain a solid tablet,

the order of steps a)-b) and c)-d) may possibly be reversed.

17. A use of a solid tablet as defined according to any of claims 9 to 15, for preparing a disinfectant solution comprising a minimum effective concentration of active chlorine.

**Fig. 1**

**Fig. 2**

**EP 3 801 650 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2008135062 A
- WO 2013083612 A
- FR 2212427